# EUROPÄISCHE PATENTSCHRIFT

(11) **EP 0 574 609 B1**
(45) Veröffentlichungstag und Bekanntmachung des Hinweises auf die Patenterteilung: **02.12.1998**
(21) Anmeldenummer: 92110294.3
(22) Anmeldetag: 17.06.1992
(51) Int. Cl.: A61N 1/39

(54) **Defibrillator/Kardiovertierer**
Defibrillator/cardioverter
Defibrillateur/cardioverteur

(43) Veröffentlichungstag der Anmeldung: 22.12.1993
(73) Patentinhaber: Pacesetter AB, 171 95 Solna (SE)
(72) Erfinder: Hedberg, Sven-Erik, S-196 32 Kungsängen (SE)
(74) Vertreter: Lettström, Richard Wilhelm

(56) Entgegenhaltungen:
- EP-A- 0 445 800
- EP-A- 0 457 604
- EP-A- 0 480 569
- DE-A- 3 236 756
- FR-A- 1 195 482
- US-A- 4 969 463
- US-A- 5 107 834

## Beschreibung

Die Erfindung betrifft einen Defibrillator/Kardiovertierer, mit einer Mehrzahl von n (n≥3) Elektroden, die an einer Einrichtung zur Erzeugung von elektrischen Impulsen angeschlossen sind.

Bei einem derartigen, aus der DE-A-39 19 498 bekannten Defibrillator oder Kardiovertierer ist eine von mehreren Elektroden im Herzinneren angeordnet, und die übrigen Elektroden sind außen am Herzen plaziert. Die äußeren Elektroden sind elektrisch miteinander verbunden und an einemen ersten von zwei Ausgangsanschlüssen des Ausgangs eines Impulsgenerators angeschlossen; die im Herzinneren angeordnete Elektrode ist mit dem zweiten Ausgangsanschluß verbunden. Damit wird erreicht, daß sich bei der Abgabe eines elektrischen Impulses durch den Impulsgenerator die elektrische Stromdichte im Herzmuskel entsprechend der Anordnung der Elektroden verteilt und dabei vorzugsweise die dicksten Zonen des Herzmuskels, die den Haupteil der Herzmuskelmasse bilden, durchdringt, um eine Defibrillation bzw. Kardioversion zu erzielen. Die Stromverteilung läßt sich nur durch die Anordnung und Größe der einzelen Elektroden einstellen, wobei jedoch die Anordnung der Elektroden und insbesondere ihr Abstand zueinander durch die anatomischen Gegebenheiten beschränkt ist.

Aus der US-A-4 548 203 ist ein weiterer Defibrillator mit mehreren Elektroden bekannt, die paarweise an unterschiedlichen Ausgängen eines Impulsgenerators angeschlossen sind und an unterschiedlichen, vorzugsweise einander gegenüberliegenden Stellen des Herzens plaziert sind. Zur Defibrillation des Herzens werden die einzelnen Elektrodenpaare nacheinander über die Ausgänge des Impulsgenerators mit jeweils einem elektrischen Impuls beaufschlagt, wobei die Impulse jeweils durch ein Zeitintervall voneinander getrennt sind. Durch die sowohl räumlich als auch zeitlich getrennte Impulsabgabe soll eine Herabsetzung der zur Auslösung der Defibrillation erforderlichen Energie erreicht werden. Da jedoch jeweils nur zwei Elektroden gleichzeitig bei der Impulsabgabe beteiligt sind, ist eine echte Verteilung der Stromdichte auf unterschiedliche Zonen des Herzmuskels kaum möglich.

Bei einem weiteren, aus den US-A-4 708 1445 bekannten Defibrillator mit drei Elektroden erfolgt die Abgabe von Defibrillierungsimpulsen sequentiell zwischen einer ersten Elektrode und jeweils einer der beiden anderen Elektroden, wobei auch hier die Impulse durch ein Zeitintervall voneinander getrennt sind.

Gemäß dem Gegenstand der älteren europäischen Patentanmeldung EP-A-505857 wird eine räumlich optimale Stromverteilung dadurch erreicht, daß die einzelnen Elektroden an ihnen jeweils zugeordneten Ausgangsanschlüssen von mehreren in Reihe geschalteten Ausgängen einer Einrichtung angeschlossen ist, die an jedem ihrer Ausgänge jeweils gleichzeitig einen elektrischen Impuls abgibt.

Der erfindung liegt die Aufgabe zugrunde, einen Defibrillator bzw. Kardiovertierer anzugeben, mit dem sich eine sowohl räumlich als auch zeitlich optimale Stromverteilung im Herzen zur Erzielung einer möglichst effektiven Defibrillation einstellen läßt.

Diese Aufgabe wird gelöst durch einem Defibrillator/Kardiovertierer, der den Kennzeichen vom Anspruch 1 aufweist.

Damit wird erreicht, daß der während der Defibrillation dem Herzen über die Elektroden zugeführte elektrische Strom in den aufeinanderfolgenden Zeitabschnitten unterschiedliche Bereiche des Herzens mit unterschiedlichen Stromdichten durchsetzt, so daß die unterschiedlichen Bereiche des Herzens nacheinander defibrilliert werden und ein Wiederaufflackern der Defibrillation verhindert wird. Durch die zeitlich überlappend erfolgende Impulsabgabe an den verschieden Ausgängen der impulserzeugenden Einrichtung können außerdem in vorgegebenen Bereichen des Herzens biphasische Stromverläufe hervorgerufen werden, die sich für Defibrillationszwecke als besonders wirksam erwiesen haben. Um die unterschiedlichen Bereiche des Herzens nacheinander mit unterschiedlichen Stromdichten beaufschlagen zu können, ist in der Erfindung vorgesehen, daß die impulserzeugende Einrichtung Mittel zur Erzeugung der Ausgangsimpulse mit jeweils unterschiedlichem Impulsbeginn, Impulsdauer und/oder Impulshöhe aufweist.

Eine besonders einfache Ausbildung des erfindungsgemäßen Defibrillators/Kardioverters wird in vorteilhafter Weise dadurch erreicht, daß die impulserzeugende Einrichtung einen Kondensator aufweist, der zum Aufladen an eine Ladeschaltung schaltbar ist, daß der Kondensator auf einer Seite über steuerbare Schalter mit jedem einzelnen der Ausgangsanschlüsse der Einrichtung verbunden ist, daß der Kondensator auf seiner anderen Seite über weitere steuerbare Schalter ebenfalls mit jedem der Ausgangsanschlüsse verbunden ist und daß eine Steuereinrichtung zum individuellen Ein- und Ausschalten der Schalter vorgesehen ist. Damit ist es möglich, zu unterschiedlichen Zeitpunkten unterschiedliche Elektroden in unterschiedlicher Zahl mit den jeweils beiden Seiten des Kondensators zu verbinden und dadurch einen gewünschten Verlauf der Stromverteilung in Herzen zu erzielen.

Entsprechend einer weiteren vorteilhaften Ausbildung des erfindungsgemäßen Defibrillators/Kardioverters ist vorgesehen, daß die impulserzeugende Einrichtung n-1 in Reihe geschaltete Kondensatoren aufweist, die zum Aufladen an eine Ladeschaltung schaltbar sind, daß die Anschlüsse der einzelnen Kondensatoren über einzeln steuerbare Schalter mit den Ausgangsanschlüssen der Einrichtung verbunden sind und daß eine Steuereinrichtung zum individuellen Ein- und Ausschalten der einzelnen Schalter vorgesehen ist. Dadurch wird eine weitere Variation der Stromverteilung im Herzen während der Defibrillierung ermöglicht, weil die Impulshöhen der dem Herzen überlappend zugeführen Impulse durch die Aufladung der Kondensatoren auf unterschiedliche Ladaspannungen eingestellt werden können. Dies kann im einfachsten Fall dadurch geschehen, daß die Reihenschaltung aus den Kondensatoren auf eine von der Ladeschaltung vorgegebene Ladespannung aufgeladen wird, wobei das Verhältnis der Teilspannungen über den einzelnen Kondensatoren von deren Kapazität abhängig ist.

Um die einzelnen Kondensatoren auf von ihrem Kapazitätsverhältnis unabhängige Teilspannungen aufladen zu können, ist in vorteilhafter Weise vorgesehen, daß die Ladeschaltung Mittel zum Aufladen der einzelnen Kondensatoren auf unterschiedliche vorgegebene Spannungen aufweist.

Entsprechend einer vorteilhaften Weiterbildung des erfindungsgemäßen Defibrillators/Kardiovertierers vorgesehen, daß an der impulserzeugenden Einrichtung ein Parameterspeicher angeschlossen ist, in dem für die unterschiedlichen Ausgangsimpulse unterschiedliche Werte für den Impulsbeginn, die Impulsdauer und/oder die Impulshöhe abgespeichert sind, und daß der Parameterspeicher mit einer Telemetrieeinrichtung zur Übertragung der Werte von einem Programmiergerät in den Parameterspeicher verbunden ist. Damit besteht insbesondere bei implantierbaren Geräten die Möglichkeit, die die Stromverteilung im Falle einer Defibrillation bestimmenden Parameterwerte von einem externen Programmiergerät in das implantierbare Gerät zu übertragen.

Da die Stromverteilung im Herzen nicht nur von den oben angegebenen Parameterwerten sondern auch der Anordnung der Elektroden und der Geometrie des Herzens abhängig ist, ist es von besonderem Vorteil, den Einfluß der Elektrodenanordnung und der Herzgeometrie auf die Stromverteilung im voraus bestimmen zu können. In diesem Zusammenhang ist in vorteilhafter Weise eine Meßeinrichtung zur Messung der elektrischen Impedanz zwischen den Elektroden vorgesehen. Alternativ hierzu kann eine Meßeinrichtung zur Messung eines elektrischen Stroms in dem Stromweg zumindest eines Ausgangsanschlusses der impulserzeugenden Einrichtung vorgesehen sein, wobei dann von der impulserzeugenden Einrichtung ein Meßimpuls an das Herz abgegeben werden kann und aus dem dadurch verursachten Strom auf die Elektrodenanordnung und Herzgeometrie zurückgeschlossen werden kann.

In vorteilhafter Weise ist die Meßeinrichtung mit der Telemetrieeinrichtung zur Übertragung der Meßwerte an das Programmiergerät verbunden. Die Meßwerte können dann beispielsweise auf einer Anzeigeeinrichtung des Programmiergeräts graphisch dargestellt werden und der Bedienperson Hinweise zur Programmierung der Parameterwerte (Impulsbeginn, Impulsdauer und Impulshöhe) geben, um eine gewünschte Stromverteilung im Herzen zu erreichen.

Entsprechend einer besonders vorteilhaften Weiterbildung des erfindungsgemäßen Defibrillators/Konvertierers ist vorgesehen, daß die Meßeinrichtung ausgangsseitig über eine Steuerleitung mit den Mitteln zur automatischen Einstellung des Impulsgbeginns, der Impulsdauer und/oder der Impulshöhe verbunden ist. Auf diese Weise wird durch den Defibrillator automatisch die jeweilige Anordnung der Elektroden und Herzgeometrie bei der Bestimmung der Parameterwerte berücksichtigt, so daß eine vorgegebene definierte Stromverteilung im Herzen erreicht wird.

Zur Erläuterung der Erfindung wird im folgenden auf die Figuren der Zeichung Bezug genommen. Im einzelnen zeigen
- FIG 1: das Blockschaltbild eines Ausführungsbeispiels des erfindungsgemäßen Defibrillators/Konvertierers,
- FIG 2: ein Ausführungsbeispiel der in dem Blockschaltbild nach Figur 1 gezeigen impulserzeugenden Einheit,
- FIG 3: ein Diagramm mit den Schaltzeiten der in Figur 2 gezeigten steuerbaren Schalter,
- FIG 4: A bis F die mit dem Defibrillator nach den Figuren 1 und 2 erzielte Stromverteilung im Herzen,
- FIG 5: ein weiteres Ausführungsbeispiel für den erfindungsgemäßen Defibrillator/Konvertierer,
- FIG 6: in einem Diagramm die Schaltzeiten für die in Figur 5 gezeigten steuerbaren Schalter und die daraus resultierenden Stromverläufe an den Ausgängen des in Figur 5 gezeigten Defibrillators und
- FIG 7: ein alternatives Ausführungsbeispiel für die bei dem Defibrillator nach Figur 5 vorhandene Meßeinrichtung.

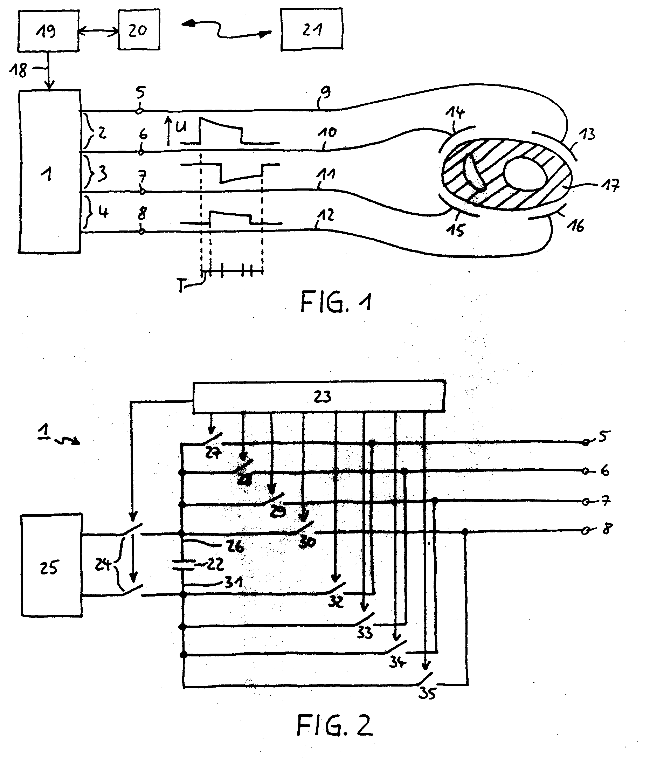

Bei dem in Figur 1 gezeigten Blockschaltbild eines Defibrillators oder Konvertierers ist mit 1 eine Einrichtung zur Erzeugung elektrischer Impulse bezeichnet. Die Einrichtung 1 weist drei in Reihe geschaltete Ausgänge 2,3 und 4 auf, deren insgesamt vier Ausgangsanschlüsse 5,6,7 und 8 über Elektrodenleitungen 9,10,11 und 12 mit vier Elektroden 13,14,15 und 16 verbunden sind, die an einem hier im Querschnitt dargestellten Herzen 17 angeordnet sind. Die Einrichtung 1 zur Erzeugung elektrischer Impulse ist über eine Steuerleitung 18 mit einem Parameterspeicher 19 verbunden, in dem unterschiedliche Werte für den jeweiligen Impulsbeginn, die Impulsdauer und gegebenenfalls die Impulshöhe der von der Einrichtung 1 an ihren Ausgängen 2,3 und 4 zu erzeugenden Impulse abgespeichert sind. Diese Werte können mittels einer an dem Parameterspeicher 19 angeschlossenen Telemetrieeinrichtung 20 zwischen dem Parameterspeicher 19 und einem Programmiergerät 21 übertragen werden. Eine derarte telemetrische Kommunikation ist insbesondere dann von Vorteil, wenn es sich bei dem in Figur 1 gezeigten Defibrillator um ein implantierbares Gerät handelt; im übrigen kann es sich bei dem Ausführungsbeispiel auch um ein nicht implantierbares Gerät handeln. Dementsprechend können die Elektroden 13,14,15 und 16 wie in Figur 1 gezeigt direkt am Herzen 17, aber auch einzeln intrakardiell, subkutan oder außen am Körper des Patienten angeordnet sein. Ferner kann die Anzahl der verwendeten Elektroden allgemein n (n≥3) betragen, wobei die Einrichtung 1 zur Erzeugung elektrischer Impulse n-1 Ausgänge mit n Ausgangsanschlüssen aufweist.

Zum Defibrillieren des Herzens 17 erzeugt die Einrichtung 1 an ihren Ausgängen 2,3 und 4 elektrische Spannungsimpulse U, die sich, wie in Figur 1 schematisch angedeutet ist, zeitlich überlappen, wobei in unmittelbar aufeinanderfolgenden Zeitabschnitten T jeweils eine unterschiedliche Anzahl von Impulsen U gleichzeitig abgegeben wird. Dies wird dadurch erreicht, daß die Impulsabgabe entsprechend den in dem Parameterspeicher 18 enthaltenen Werten für die einzelnen Ausgänge 2,3 und 4 mit unterschiedlichen Zeiten für den jeweiligen Impulsbeginn und die jeweilige Impulsdauer erfolgt. Welchen Einfluß dies auf die Defibrillation des Herzens 17 hat, wird im folgenden an Hand weiterer Ausführungsbeispiele erläutert.

Figur 2 zeigt ein besonderes einfaches Ausführungsbeispiel für die impulserzeugende Einheit 1, bei dem ein Kondensator 22 zum Aufladen auf eine vorgegebene Spannung über eine von einer Steuereinrichtung 23 gesteuerte Schalteranordnung 24 an eine Ladeschaltung 25 schaltbar ist. Der Kondensator 22 ist auf einer Seite 26 über vier steuerbare Schalter 27 bis 30 mit jedem einzelnen der Ausgänge 5 bis 8 verbunden und auf der anderen Seite 31 über vier weitere steuerbare Schalter 32 bis 35 ebenfalls mit jedem der Ausgänge 5 bis 8 verbunden. Die Schalter 27 bis 30 und 32 bis 35 sind unabhängig voneinander einzeln durch die Steuereinrichtung 23 ein- und auschaltbar. An den Ausgangsanschlüssen 5 bis 8 sind, wie in Figur 1 gezeigt, vier Elektroden angeschlossen.

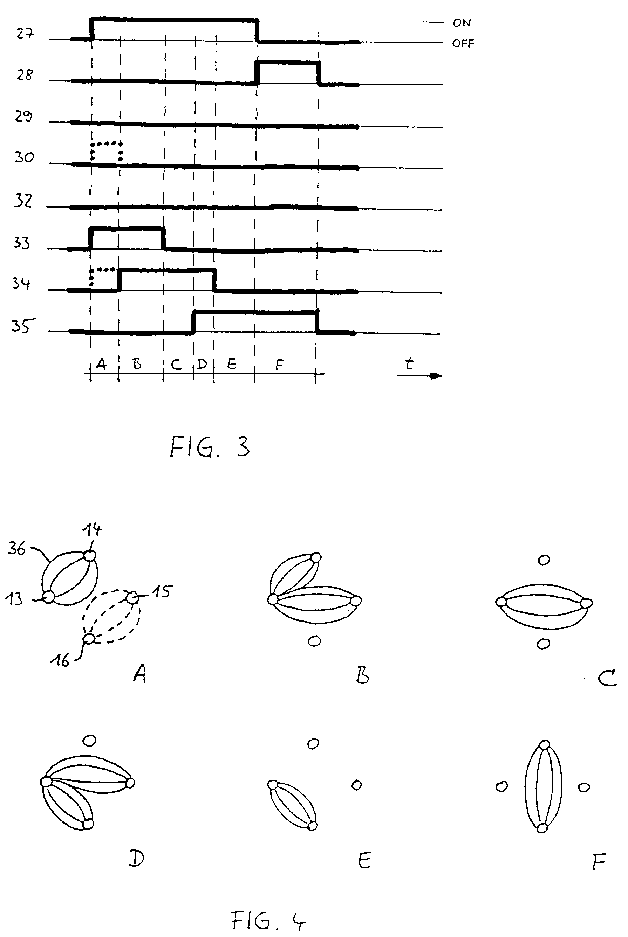

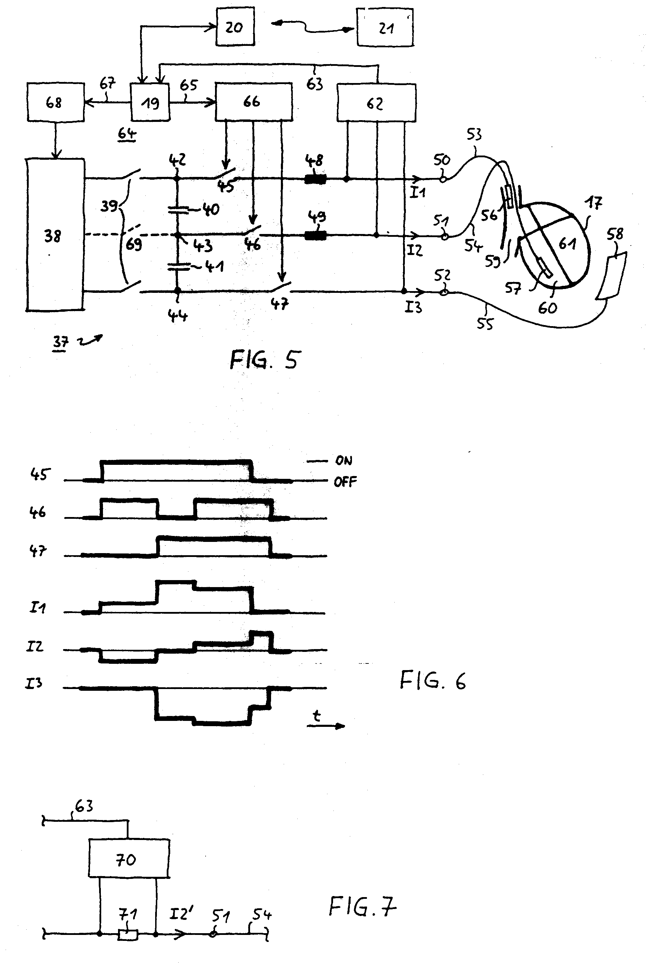

In Figur 3 ist einem Diagramm ein Beispiel für die Ansteuerung der Schalter 27 bis 30 und 32 bis 35 während zusammenhängender, unmittelbar aufeinanderfolgender Zeitabschnitte A bis F dargestellt. Figur 4 zeigt die daraus resultierende Stromverteilung 36 zwischen den Elektroden 13,14,15 und 16 während der einzelnen Zeitabschnitte A-F.

Wie Figur 4 für die verschiedenen Zeitabschnitte A bis F zeigt, kann die Stromverteilung 36 auf unterschiedliche Bereiche des hier schematisch angedeuteten Herzgewebes 17 verteilt werden. Insbesondere kann, wie Figur 4 zeigt, die Steuerung der Stromverteilung 36 in der Weise erfolgen, daß die Stromverteilung 36 in den aufeinanderfolgenden Zeitabschnitten A bis F nacheinander jeweils benachbarte Bereiche des Herzgewebes 17 durchsetzt, so daß ähnlich einem konzentrierten Wasserstrahl, der kontinuierlich über einen Brandherd geführt wird, die unterschiedlichen Bereiche des Herzgewebes 17 nacheinander defibrilliert werden. Dabei kann durch die Wahl der Schaltfolge für die steuerbaren Schalter 27 bis 30 und 32 bis 35 die Defibrillation des Herzgewebes 17 zunächst auf den von der Fibrillation am stärksten betroffenen Bereich des Herzgewebes 17 konzentriert werden und anschließend auf Randbereiche des Herzgewebes 17 ausgedehnt werden oder umgekehrt beginnend mit der Defibrillation von geringer betroffenen Bereichen des Herzgewebes 17 auf den am stärksten betroffenen Bereich des Herzgewebes 17 konzentriert werden. Dieser Vorgang kann bei Bedarf auch mehrmals wiederholt werden. Dadurch wird erreicht, daß die einzelnen Bereiche des Herzgewebes 17 nacheinander defibrilliert werden und ein Wiederaufflackern der Fibrillation in diesen Bereichen verhindert wird.

Bei dem in Figur 5 gezeigten Ausführungsbeispiel für einen implantierbaren Defibrillator 37 ist eine Ladeschaltung 38 über eine steuerbare Schalteranordnung 39 an zwei in Reihe liegende Kondensatoren 40 und 41 schaltbar. Die Reihenschaltung der Kondensatoren 40 und 41 weist drei unterschiedliche Anschlußstellen 42,43 und 44 auf, die jeweils über unabhängig voneinander steuerbare Schalter 45,46 und 47 sowie im Falle der mit 42 und 43 bezeichneten Anschlußstelle über Induktivitäten 48 und 49 mit Ausgangsanschlüssen 50,51 und 52 des Defibrillators 37 verbunden sind. Entsprechend der Anzahl der in Reihe liegenden Kondensatoren 40 und 41 bilden die Ausgangsanschlüsse 50,51 und 52 zwei in Reihe liegende Ausgänge des Defibrillators 37, die über Elektrodenleitungen 53,54 und 55 mit Elektroden 56,57 und 58 verbunden sind. Dabei ist die Elektrode 56 in der vena cava 59, die Elektrode 57 im rechten Ventrikel 60 und Elektrode 58 dem linken Ventrikel 61 des Herzens 17 gegenüberliegend vorzugsweise subkutan angeordnet. An den Ausgangsanschlüssen 50,51 und 52 ist eine Impedanzmeßeinrichtung 62 angeschlossen, die zu vorgegebenen Zeitpunkten, vorzugsweise unmittelbar vor dem Auslösen einer Defibrillation des Herzens 17, die elektrische Impedanz des Herzgewebes 17 zwischen den Elektroden 56,57 und 58 mißt. Das in der Meßeinrichtung 62 ausgewertete Impedanzmeßsignal wird über eine Steuerleitung 63 einer Einrichtung 64 zur automatischen Einstellung des jeweiligen Impulsbeginns, der Impulsdauer und der Impulshöhe für die von dem Defibrillator 37 an das Herz 17 abzugebenden elektrischen Impulse zugeführt. Die Einrichtung 64 besteht aus einem eingangsseitig an der Steuerleitung 63 angeschlossenen Parameterspeicher 19, der über einen ersten Parameterausgang 65 mit einer Steuereinrichtung 66 zum individuellen Ein- und Ausschalten der Schalter 45,46 und 47 verbunden ist und über einen zweiten Parameterausgang 67 an einer weiteren Steuereinrichtung 68 zum Einstellen der von der Ladeschaltung 38 zum Aufladen der Kondensatoren 40 und 41 erzeugten Ladespannung angeschlossen ist. Die in dem Parameterspeicher 19 enthaltenen Werte für den Impulsbeginn, die Impulsdauer und die Impulshöhe der zu erzeugenden Impulse können mittels einer an dem Parameterspeicher 19 angeschlossenen Telemetrieeinrichtung 20 an ein externes Programmiergerät 21 zur Information einer Bedienperson übertragen werden. Darüberhinaus können die Parameterwerte auch von der Bedienperson über das Programmiergerät 21 in den Parameterspeicher 19 eingegeben werden, oder es können Randbedingungen durch die Bedienperson programmiert werden, unter denen die automatische Parametereinstellung durch die Meßeinrichtung 62 erfolgt.

Die Ladeschaltung 38 erzeugt eine einzige Ladespannung, die bei geschlossener Schalteranordnung 39 über den in Reihe liegenden Kondensatoren 40 und 41 liegt und diese auf von den Kapazitäten der Kondensatoren 40 und 41 abhängige Teilspannungen auflädt. Alternativ hierzu können die Kondensatoren 40 und 41 auf vom Verhältnis ihrer Kapazitätswerte unabhängige Teilspannungen aufgeladen werden, wozu die Ladeschaltung 48 für jeden Kondensator 40 und 41 jeweils eine Ladespannung liefert und die beiden unterschiedlichen Ladespannungen über die um einen zusätzlichen, hier gestrichelt dargestellten Schalter 69 erweiterte steuerbare Schalteranordnung 38 an die Kondensatoren 40 und 41 geschaltet werden.

Solange also die Schalteranordnung 39 geschlossen ist, werden die Kondensatoren 40 und 41 auf die von der Ladeschaltung 38 vorgegebene Ladespannung aufgeladen, wobei die Teilspannungen über den einzelnen Kondensatoren 40 und 41 von ihrem Kapazitätsverhältnis abhängig ist oder direkt von der Ladeschaltung 38 eingestellt wird. Mit den unterschiedlichen Teilspannungen wird der Tatsache Rechnung getragen, daß die Herzmuskelmasse in bezug auf die gewählte Anordnung der Elektroden 56,57 und 58 ungleichmäßig verteilt ist. Zur Defibrillation des Herzens 17 werden die Schalter 45,46 und 47 geschlossen, so daß sich die Kondensatoren 40 und 41 über die Induktivitäten 48 und 49 sowie das zwischen den Elektroden 56,57 und 58 liegende Herzgewebe 17 entladen, wobei dem Herzen 17 über die Elektroden 56,57 und 58 unterschiedliche Stromimpulse I1, I2 und I3 zugeführt werden. Durch die Induktivitäten 48 und 49 wird die Steilheit der Anstiegs- und Abfallflanken der Stromimpulse I1,I2 und I3 begrenzt, wobei bei entsprechend hohen Induktivitätswerten die Stromimpulse I1,I2 und I3 die Form von stark gedämpften Sinusschwingungen erhalten können.

Die im Herzen 17 während der Defibrillation erreichte Stromverteilung ist außer von der Geometrie des Herzmuskels und der Anordnung der Elektroden 56,57 und 58 auch von den gewählten Teilspannungen über den Kondensatoren 40 und 41 und den Schaltzeiten für die Schalter 45,46 und 47 abhängig. Dabei wird der Einfluß der Geometrie des Herzens 17 und der Anordnung der Elektroden 56,57 und 58 auf die Stromverteilung vor dem Auslösen der Defibrillation durch Messen der Impedanz zwischen den Elektroden 56,57 und 58 ermittelt und zur Einstellung der Werte für die Teilspannungen an den Kondensatoren 40 und 41 und die Schaltzeiten für die Schalter 45,46 und 47 herangezogen. Dadurch wird eine vorherbestimmte definierte Verteilung des Defibrillierungsstromes im Herzen 17 erreicht.

In Figur 6 ist in einem Diagramm ein Beispiel für die Ansteuerung der Schalter 45,46 und 47 und der daraus resultierende Verlauf der Ströme I1,I2 und I3 dargestellt. Dabei wurde ein Verhältnis der Teilspannungen über den Kondensatoren 40 und 41 von 1 : 2 angenommen. Wie Figur 6 zeigt, können aufgrund der sich unterschiedlich überlappenden Schaltzeiten für die Schalter 45,46 und 47 unterschiedliche, im Falle des Stromes I2 sogar gezielt biphasische Stromverläufe I1,I2 und I3 erreicht werden. Die in Figur 6 gezeigten Stromverläufe sind nur als ein Beispiel für die Erläuterung der Erfindung zu verstehen.

Figur 7 zeigt als Alternative zu der in Figur 5 gezeigten Impedanzmeßeinrichtung 22 eine Meßeinrichtung 70 zur Erfassung eines Meßstroms I2' in der Elektrodenleitung 54, wobei durch die Meßeinrichtung 70 der in einem Meßwiderstand 71 durch den Meßstrom I2' hervorgerufene Spannungsabfall gemessen wird. Der Meßwert wird über die Steuerleitung 63 zur Einstellung der Parameterwerte in dem Parameterspeicher 19 herangezogen. Der Strom I2' wird vor der Defibrillation des Herzens 17 erzeugt, indem die Kondensatoren 40 und 41 über die Ladeschaltung 38 auf eine im Vergleich zu für Defibrillationszwecke verwendete Spannungen niedrige Ladespannung aufgeladen werden und über die steuerbaren Schalter 45 bis 47 und die Elektroden 56,57 und 58 an das Herzgewebe 17 geschaltet werden.

### Bezugszeichenliste

- 1: impulserzeugende Einrichtung
- 2,3,4: Ausgänge von 1
- 5,6,7,8: Ausgangsanschlüsse von 1
- 9,10,11,12: Elektrodenleitungen
- 13,14,15,16: Elektroden
- 17: Herz
- 18: Steuerleitung
- 19: Parameterspeicher
- 20: Telemetrieeinrichtung
- 21: Programmiergerät
- 22: Kondensator
- 23: Steuereinrichtung
- 24: Schalteranordnung
- 25: Ladeschaltung
- 26: eine Seite von 22
- 27,28,29,30: steuerbare Schalter
- 31: andere Seite von 22
- 32,33,34,35: weitere steuerbare Schalter
- 36: Stromverteilung in 17
- 37: Defibrillator
- 38: Ladeschaltung
- 39: Schalteranordnung
- 40,41: Kondensatoren
- 42,43,44: Anschlußstellen von 40,41
- 45,46,47: steuerbare Schalter
- 48,49: Induktivitäten
- 50,51,52: Ausgangsanschlüsse von 37
- 53,54,55: Elektrodenleitungen
- 56,57,58: Elektroden
- 59: vena cava
- 60: rechter Ventrikel
- 61: linker Ventrikel
- 62: Impedanzmeßeinrichtung
- 63: Steuerleitung
- 64: Einrichtung zur automatischen Einstellung von Impulsbeginn, -dauer und -höhe
- 65: erster Parameterausgang von 19
- 66: Steuereinrichtung
- 67: zweiter Parameterausgang von 19
- 68: weitere Steuereinrichtung
- 69: Schalter
- 70: Strommeßeinrichtung
- 71: Meßwiderstand
- A-F: Zeitabschnitte
- I1-I3,I2': Ströme
- T: Zeitabschnitt
- U: Spannungsimpuls

## Patentansprüche

1. Defibrillator/Kardiovertierer mit einer Mehrzahl vonn, n ≥ 3, Elektroden (13-16, 56-58), die an einer Einrichtung (1) zur Erzeugung von elektrischen Impulsen (U) angeschloßen sind, welche Einrichtung (1) n - 1 in Reihe geschaltete Ausgänge (2-4) aufweist, an deren insgesamt n Ausgangsanschlüßen (5-8, 50-52) mit den Elektroden (13-16, 56-58) über Elektrodenzuleitungen angeschloßen sind, und wobei die impulserzeugende Einrichtung (1) an jedem ihrer Ausgänge (2-4) jeweils einen elektrischen Ausgangsimpuls abgibt, **dadurch gekennzeichnet**, daß die Einrichtung (1) einen Parameterspeicher (19) aufweist, in dem unterschiedliche Werte für den jeweiligen Impulsbeginn, Impulsdauer und/oder Impulshöhe der Ausgangsimpulse abgespeichert sind, und daß die Einrichtung (1) steuerbare Schalter (27-30, 32-35, 45, 46, 47) aufweist, die die Ausgänge ein- und ausschalten, und die unter Steuerung von dem Parameterspeicher (19) in eine Schaltfolge derart angesteuert werden, daß in unmittelbar aufeinanderfolgenden Zeitabschnitten jeweils eine unterschiedliche Anzahl der Ausgangsimpulse gleichzeitig abgegeben wird, so daß die Ausgangsimpulse sich zeitlich überlappen.

2. Defibrillator/Kardiovertierer nach Anspruch 1, **dadurch gekennzeichnet**, daß die impulserzeugende Einrichtung (1) einen Kondensator (22) aufweist, der zum Aufladen an eine Ladeschaltung (25) schaltbar ist, daß der Kondensator (22) auf einer Seite (26) über eine Anzahl der steuerbaren Schalter (27-30) mit jedem einzelnen der Ausgangsanschlüsse (5-8) der Einrichtung (1) verbunden ist, daß der Kondensator (22) auf seiner anderen Seite (31) über eine weitere Anzahl der steuerbaren Schalter (32-35) ebenfalls mit jedem der Ausgangsanschlüsse (5-8) verbunden ist und daß eine Steuerinrichtung (23) zum individuellen Ein- und Ausschalten der Schalter (27-30 und 32-35) vorgesehen ist.

3. Defibrillator/Kardiovertierer nach Anspruch 1, **dadurch gekennzeichnet**, daß die impulserzeugende Einrichtung (1) n-1 in Reihe geschaltete Kondensatoren (40, 41) aufweist, die zum Aufladen an eine Ladeschaltung (38) schaltbar sind, daß die Anschlüsse (42, 43, 44) der einzelnen Kondensatoren (40, 41) über einzeln der steuerbaren Schalter (45, 46, 47) mit den Ausgangsanschlüssen (50, 51, 52) der Einrichtung (1) verbunden sind und daß eine Steuerinrichtung (66) zum individuellen Ein- und Ausschalten der einzelnen Schalter (45, 46, 47) vorgesehen ist.

4. Defibrillator/Kardiovertierer nach Anspruch 3, **dadurch gekennzeichnet**, daß die Ladeschaltung (38) Mittel (68) zur Aufladung der einzelnen Kondensatoren (40, 41) auf unterschiedliche vorgegebene Spannungen aufweist.

5. Defibrillator/Kardioverierer nach einem der Ansprüche 1 bis 4, **dadurch gekennzeichnet**, daß in dem Parameterspeicher (19) für die unterschiedlichen Ausgangsimpulse (U) unterschiedliche Werte für den Impulsbeginn, die Impulsdauer und/oder die Impulshöhe abgespeichert sind und daß der Parameterspeicher (19) mit einer Telemetrieeinrichtung (20) zur Übertragung der Werte von einem Programmiergerät (21) in den Parameterspeicher (19) verbunden ist.

6. Defibrillator/Kardiovertierer nach einem der vorangehenden Ansprüche, **dadurch gekennzeichnet**, daß eine Meßeinrichtung (62) zur Messung der elektrichen Impedanz zwischen den Elektroden (56, 57, 58) vorgesehen ist.

7. Defibrillator/Kardiovertierer nach einem vorangehenden Ansprüche, **dadurch gekennzeichnet**, daß eine Meßeinrichtung (70) zur Messung eines elektrischen Stroms (I2') in dem Stromweg zumindest eines Ausgangsanschlusses (51) der impulserzeugenden Einrichtung (1) vorgesehen ist.

8. Defibrillator/Kardiovertierer nach Anspruch 5 und 6 oder 5 und 7, **dadurch gekennzeichnet**, daß die Meßeinrichtung (62, 70) mit der Telemetrieeinrichtung (20) zur Übertragung der Meßwerte an das Programmiergerät (21) verbunden ist.

9. Defibrillator(Kardiovertierer nach einem der Ansprüche 6 bis 8 **dadurch gekennzeichnet**, daß die Meßeinrichtung (62, 70) ausgangsseitig über eine Steuerleitung (63) mit den Mitteln (64; 19, 66, 68) zur automatiscen Einstellung des Impulsbeginns, der Impulsdauer und/oder der Impulshöhe verbunden ist.

## Claims

1. Defibrillator/cardioverter having a plurality of n, n≥3, electrodes (13-16, 56-58) which are connected to a device (1) for generating electrical pulses (U), which device (1) has n - 1 series-connected outputs (2-4), the output terminals (5-8, 50-52) of which, which number n in total, are connected by way of electrode supply lines to the electrodes (13-16, 56-58), and with the pulse-generating device (1) emitting at each of its outputs (2-4) a respective electrical output pulse, characterized in that the device (1) has a parameter memory (19) in which different values for the respective pulse beginning, pulse duration and/or pulse height of the output pulses are stored, and in that the device (1) has controllable switches (27-30, 32-35, 45, 46, 47) which connect and disconnect the outputs, and which are activated with control by the parameter memory (19) into a switching sequence in such a way that in immediately successive time segments a respective different number of output pulses is simultaneously emitted so that the output pulses overlap temporally.

2. Defibrillator/cardioverter according to claim 1, characterized in that the pulse-generating device (1) has a capacitor (22) which can be connected to a charging circuit (25) for charging, in that the capacitor (22) is connected on one side (26) by way of a number of the controllable switches (27-30) to each individual output terminal (5-8) of the device (1), in that the capacitor (22) is connected on its other side (31) by way of a further number of the controllable switches (32-35) likewise to each of the output terminals (5-8) and in that a control device (23) is provided for the individual connection and disconnection of the switches (27-30 and 32-35).

3. Defibrillator/cardioverter according to claim 1, characterized in that the pulse-generating device (1) has n-1 series-connected capacitors (40, 41) which can be connected to a charging circuit (38) for charging, in that the connections (42, 43, 44) of the individual capacitors (40, 41) are connected by way of individual controllable switches (45, 46, 47) to the output terminals (50, 51, 52) of the device (1) and in that a control device (66) is provided for the individual connection and disconnection of the individual switches (45, 46, 47).

4. Defibrillator/cardioverter according to claim 3, characterized in that the charging circuit (38) has means (68) for charging the individual capacitors (40, 41) to different specified voltages.

5. Defibrillator/cardioverter according to one of claims 1 to 4, characterized in that in the parameter memory (19) for the different output pulses (U) different values for the pulse beginning, the pulse duration and/or the pulse height are stored, and in that the parameter memory (19) is connected to a telemetry device (20) for the transfer of the values from a programming device (21) into the parameter memory (19).

6. Defibrillator/cardioverter according to one of the preceding claims, characterized in that a measuring device (62) is provided for measuring the electrical impedance between the electrodes (56, 57, 58).

7. Defibrillator/cardioverter according to one of the preceding claims, characterized in that a measuring device (70) is provided for measuring an electric current (I2') in the current path of at least one output terminal (51) of the pulse-generating device (1).

8. Defibrillator/cardioverter according to claim 5 and 6 or 5 and 7, characterized in that the measuring device (62, 70) is connected to the telemetry device (20) for the transfer of the measured values to the programming device (21).

9. Defibrillator/cardioverter according to one of claims 6 to 8, characterized in that the measuring device (62, 70) is connected on the output side by way of a control line (63) to the means (64; 19, 66, 68) for the automatic setting of the pulse beginning, pulse duration and/or pulse height.

## Revendications

1. Défibrillateur/cardioverteur, comportant une pluralité de n électrodes (n≥3) (13 à 16, 56 à 58), qui sont raccordées à un dispositif (1) servant à produire des impulsions électriques (U), lequel dispositif (1) présente n-1 sorties (2 à 4) montées en série et au niveau desquelles au total n bornes de sortie (5 à 8, 50 à 52) sont raccordées aux électrodes (13 à 16, 56 à 58) par l'intermédiaire de lignes d'alimentation d'électrodes, et dans lequel le dispositif générateur d'impulsions (1) fournit, à chacune de ses sorties (2 à 4), chaque fois une impulsion électrique de sortie, caractérisé par le fait que le dispositif (1) présente une mémoire de paramètres (19), dans laquelle sont mémorisées des valeurs différentes pour chaque fois le début des impulsions, la durée des impulsions et/ou l'amplitude des impulsions, pour les impulsions de sortie, et que le dispositif (1) présente des interrupteurs (27 à 30, 32 à 35, 45, 46, 47) pouvant être commandés et mettant en et hors fonction les sorties, tandis qu'ils sont attaqués, sous la commande de la mémoire de paramètres (19) selon une séquence de commutation telle que, dans des intervalles de temps directement successifs, il est chaque fois fourni simultanément un nombre différent des impulsions de sortie, de telle sorte que les impulsions de sortie soient à chevauchement temporel.

2. Défibrillateur/cardioverteur selon la revendication 1, caractérisé par le fait que le dispositif générateur d'impulsions (1) comporte un condensateur (22), qui peut être commuté, pour la charge, sur un circuit de charge (25), que le condensateur (22) est relié par un côté (26), par l'intermédiaire d'un certain nombre d'interrupteurs (27 à 30) pouvant être commandés, individuellement à chacune des bornes de sortie (5 à 8) du dispositif (1), que le condensateur (22) est également relié, par son autre côté (31), par l'intermédiaire d'un autre nombre d'interrupteurs (32 à 35) pouvant être commandés, à chacune des bornes de sortie (5 à 8), et qu'il est prévu un dispositif de commande (23) pour mettre individuellement en et hors fonction les interrupteurs (27 à 30 et 32 à 35).

3. Défibrillateur/cardioverteur selon la revendication 1, caractérisé par le fait que le dispositif générateur d'impulsions (1) présente n-1 condensateurs (40, 41) montés en série, qui peuvent être commutés pour la charge sur un circuit de charge (38), que les bornes (42, 43, 44) des condensateurs (40, 41) individuels sont reliées, par l'intermédiaire d'interrupteurs individuels des interrupteurs (45, 46, 47) pouvant être commandés, aux bornes de sortie (50, 51, 52) du dispositif (1), et qu'il est prévu un dispositif de commande (66) pour la mise en et hors fonction individuellement des interrupteurs (45, 46, 47) individuels.

4. Défibrillateur/cardioverteur selon la revendication 3, caractérisé par le fait que le circuit de charge (38) présente des moyens (68) servant à charger les condensateurs (40, 41) individuels à des tensions prédéfinies différentes.

5. Défibrillateur/cardioverteur selon l'une des revendications 1 à 4, caractérisé par le fait que sont mémorisées, dans la mémoire de paramètres (19), différentes valeurs pour les différentes impulsions de sortie (U), quant au début des impulsions, quant à la durée des impulsions et/ou l'amplitude des impulsions, et que la mémoire de paramètres (19) est reliée à un dispositif de télémesure (20) pour la transmission des valeurs depuis un appareil de programmation (21) jusque dans la mémoire de paramètres (19).

6. Défibrillateur/cardioverteur selon l'une des revendications précédentes, caractérisé par le fait qu'il est prévu un dispositif de mesure (62) servant à mesurer l'impédance électrique entre les électrodes (56, 57, 58).

7. Défibrillateur/cardioverteur selon l'une des revendications précédentes, caractérisé par le fait qu'il est prévu un dispositif de mesure (70) destiné à mesurer un courant électrique (I2') dans le trajet du courant d'au moins une borne de sortie (51) du dispositif générateur d'impulsions (1).

8. Défibrillateur/cardioverteur selon les revendications 5 et 6 ou 5 et 7, caractérisé par le fait que le dispositif de mesure (62, 70) est relié au dispositif de télémesure (20) pour la transmission des valeurs de mesure à l'appareil de programmation (21).

9. Défibrillateur/cardioverteur selon l'une des revendications 6 à 8, caractérisé par le fait que le dispositif de mesure (62, 70) est relié, du côté sortie, par l'intermédiaire d'une ligne de commande (63), aux moyens (64, 19, 66, 68) servant au réglage automatique du début des impulsions, de la durée des impulsions et/ou de l'amplitude des impulsions.
